(19) Europäisches Patentamt / European Patent Office / Office européen des brevets

(11) **EP 4 424 729 A1**

(12) **EUROPEAN PATENT APPLICATION**
published in accordance with Art. 153(4) EPC

(43) Date of publication:
**04.09.2024  Bulletin 2024/36**

(21) Application number: **22887043.2**

(22) Date of filing: **26.10.2022**

(51) International Patent Classification (IPC):
***C08F 222/02*** (2006.01)

(52) Cooperative Patent Classification (CPC):
**C08F 222/02**

(86) International application number:
**PCT/JP2022/039822**

(87) International publication number:
**WO 2023/074718 (04.05.2023 Gazette 2023/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.10.2021  JP 2021175458**

(71) Applicant: **Sumitomo Seika Chemicals Co., Ltd.
Kako-gun, Hyogo 675-0145 (JP)**

(72) Inventors:
- **NOGUCHI, Mari
  Himeji-shi, Hyogo 672-8076 (JP)**
- **CHUGO, Shohei
  Himeji-shi, Hyogo 672-8076 (JP)**
- **HAYASHI, Yoshiki
  Himeji-shi, Hyogo 672-8076 (JP)**
- **NAKATSUKA, Akio
  Himeji-shi, Hyogo 672-8076 (JP)**

(74) Representative: **Vossius & Partner
Patentanwälte Rechtsanwälte mbB
Siebertstrasse 3
81675 München (DE)**

(54) **CROSSLINKED POLYMER OF ALPHA,BETA-UNSATURATED CARBOXYLIC ACID COMPOUND, AND APPLICATION OF SAME**

(57)  Provided is a polymer of a water-soluble ethylenically unsaturated monomer that can be used in preparing an external composition that is less sticky and gives a refreshing sensation and lightness in use, while also achieving an excellent moist texture and ease of blending into the skin (ease of spreading) in use. More specifically, provided is a crosslinked polymer of an $\alpha,\beta$-unsaturated carboxylic acid compound, the polymer being crosslinked by a crosslinking agent, wherein the crosslinked polymer has a ratio of S1 to S2 (S1/S2) within the range of 1.0 to 1.6 as calculated according to the following formulas: $S1 = (k[\eta]_{aq}/k[\eta]_{BG})^{1/3}$, and $S2 = (\text{particle size}_{aq}/\text{particle size}_{BG})$ wherein $k[\eta]_{BG}$ represents a product of interaction coefficient $k_{BG}$ and intrinsic viscosity $[\eta]_{BG}$, as determined by measuring a solution of the crosslinked polymer in 1,3-butylene glycol having an initial concentration of 0.3 mass% with an Ubbelohde dilution viscometer, $k[\eta]_{aq}$ represents a product of interaction coefficient $k_{aq}$ and intrinsic viscosity $[\eta]_{aq}$, as determined by measuring a neutralized aqueous solution of the crosslinked polymer having an initial concentration of 0.015 mass% with an Ubbelohde dilution viscometer, particle size$_{BG}$ represents a volume average particle size (pm) of the crosslinked polymer in a 0.3 mass% 1,3-butylene glycol solution, and particle size$_{aq}$ represents a volume average particle size ($\mu$m) of the crosslinked polymer in a 0.1 mass% neutralized aqueous solution.

**Description**

Technical Field

The present disclosure relates to a specific crosslinked polymer of an $\alpha,\beta$-unsaturated carboxylic acid compound and the use of the crosslinked polymer.

Background Art

Polymers of a water-soluble ethylenically unsaturated monomer are used as hydrophilic thickeners. For example, such polymers are preferably used in improving the viscosity of external preparation compositions in the pharmaceutical and cosmetic fields.

Citation List

Patent Literature

PTL 1: Patent No. 4883844
PTL 2: Patent No. 5943798
PTL 3: Patent No. 6182975
PTL 4: Patent No. 4630101

Summary of Invention

Technical Problem

However, external compositions containing a polymer of a water-soluble ethylenically unsaturated monomer are extremely sticky, which makes it difficult for users to experience a refreshing sensation or lightness in use. On the other hand, suppressing the stickiness could reduce the moist texture of the composition and may make it hard for the composition to seamlessly blend into (or easily spread on) the skin in use. Although several studies and developments have been made to solve this problem, the problem has yet to be fully resolved.
Thus, there has been demand for a polymer of a water-soluble ethylenically unsaturated monomer that can be used in preparing an external composition that is less sticky and gives a refreshing sensation and lightness in use, while also achieving an excellent moist texture and easily blending into (or easily spreading on) the skin in use.

Solution to Problem

The inventors newly found that a crosslinked polymer of an $\alpha,\beta$-unsaturated carboxylic acid compound (i.e., a polymer of an $\alpha,\beta$-unsaturated carboxylic acid compound crosslinked by a crosslinking agent) can be homogeneously dispersed in 1,3-butylene glycol (simply "BG" below). The inventors also found that the properties of a crosslinked polymer of an $\alpha,\beta$-unsaturated carboxylic acid compound as a thickener could possibly be examined by measuring parameter R using a BG dispersion of the crosslinked polymer of an $\alpha,\beta$-unsaturated carboxylic acid compound and a neutralized aqueous solution of the crosslinked polymer of an $\alpha,\beta$-unsaturated carboxylic acid compound.
The inventors then found it preferable to set parameter R within a specific range in order to solve the problem and further found that a crosslinked polymer of an $\alpha,\beta$-unsaturated carboxylic acid compound that satisfies parameter R within the specific range can be prepared by precipitation polymerization at lower temperatures than before.
The inventors made further improvements on the basis of the findings.
The present disclosure encompasses, for example, the subject matter described in the following items.

Item 1. A crosslinked polymer of an $\alpha,\beta$-unsaturated carboxylic acid compound, the polymer being crosslinked by a crosslinking agent,
wherein

the crosslinked polymer has a ratio of S1 to S2 (S1/S2) within the range of 1.0 to 1.6 as calculated according to the following formulas :

$$S1 = (k[\eta]_{aq}/k[\eta]_{BG})^{1/3}$$

$$S2 = (\text{particle size}_{aq}/\text{particle size}_{BG})$$

wherein

$k[\eta]_{BG}$ represents a product of interaction coefficient $k_{BG}$ and intrinsic viscosity $[\eta]_{BG}$, as determined by measuring a solution of the crosslinked polymer in 1,3-butylene glycol having an initial concentration of 0.3 mass% with an Ubbelohde dilution viscometer,
$k[\eta]_{aq}$ represents a product of interaction coefficient $k_{aq}$ and intrinsic viscosity $[\eta]_{aq}$, as determined by measuring a neutralized aqueous solution of the crosslinked polymer having an initial concentration of 0.015 mass% with an Ubbelohde dilution viscometer,
particle size$_{BG}$ represents a volume average particle size ($\mu$m) of the crosslinked polymer in a 0.3 mass% 1,3-butylene glycol solution, and
particle size$_{aq}$ represents a volume average particle size ($\mu$m) of the crosslinked polymer in a 0.1 mass% neutralized aqueous solution.

Item 2. The crosslinked polymer according to Item 1, wherein a 0.5 mass% neutralized aqueous solution of the crosslinked polymer at 25°C has a yield stress of 20 Pa or lower.
Item 3. The crosslinked polymer according to Item 1 or 2, wherein the $\alpha,\beta$-unsaturated carboxylic acid compound is at least one member selected from the group consisting of acrylic acid, methacrylic acid, crotonic acid, maleic acid, fumaric acid, itaconic acid, and salts thereof.
Item 4. The crosslinked polymer according to any one of Items 1 to 3, wherein the crosslinking agent is a compound having two or more ethylenically unsaturated groups.
Item 5. The crosslinked polymer according to any one of Items 1 to 4, which is a polymer comprising 0.15 to 2 parts by mass of the crosslinking agent per 100 parts by mass of the $\alpha,\beta$-unsaturated carboxylic acid compound.
Item 6. The crosslinked polymer according to any one of Items 1 to 5, wherein S2 is 2 to 2.5.
Item 7. A thickener for preparing an external composition, the thickener comprising the crosslinked polymer of any one of Items 1 to 6.
Item 8. An external composition comprising the crosslinked polymer of any one of Items 1 to 6.
Item 9. A method for producing a polymer of an $\alpha,\beta$-unsaturated carboxylic acid compound crosslinked by a crosslinking agent, comprising reacting an $\alpha,\beta$-unsaturated carboxylic acid compound and a compound having two or more ethylenically unsaturated groups in an inert solvent at 40 to 48°C in the presence of a radical polymerization catalyst.

Advantageous Effects of Invention

Provided is a crosslinked polymer of an $\alpha,\beta$-unsaturated carboxylic acid compound that can be used in preparing an external composition that is less sticky and gives a refreshing sensation and lightness in use, while also achieving an excellent moist texture and ease of blending into the skin (ease of spreading) in use.
The crosslinked polymer is particularly suitable for cosmetic compositions and external pharmaceutical compositions among external compositions. Viscous compositions containing a crosslinked polymer of an $\alpha,\beta$-unsaturated carboxylic acid compound (e.g., a gel containing a carbomer) may also contain other organic compounds. From a safety perspective, it is not desirable for the compositions to contain such organic compounds. The crosslinked polymer of an $\alpha,\beta$-unsaturated carboxylic acid compound provided by the present disclosure can be used together with water, butylene glycol, or other safe ingredients to prepare a composition that exhibits suitable viscosity. Thus, the resulting external composition is safer and more desirable in this respect as well.

Brief Description of Drawings

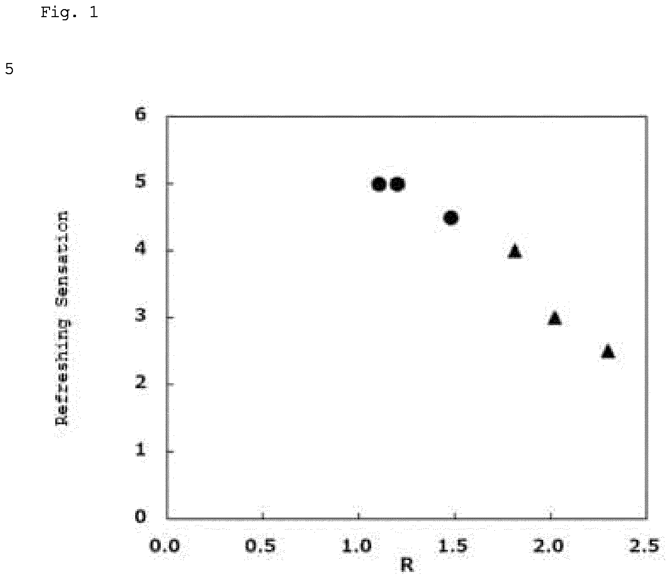

Fig. 1 is a graph showing the relation between value R and refreshing sensation.

Description of Embodiments

The following describes in more detail embodiments encompassed by the present disclosure. The disclosure preferably encompasses, but is not limited to, a crosslinked polymer of an $\alpha,\beta$-unsaturated carboxylic acid compound (i.e., a polymer of an $\alpha,\beta$-unsaturated carboxylic acid compound crosslinked by a crosslinking agent), a production method for the crosslinked polymer, use of the crosslinked polymer, an external composition containing the crosslinked polymer, etc. The disclosure encompasses all matter that is disclosed in the present specification and recognizable to those skilled in the art.

The crosslinked polymer of an $\alpha,\beta$-unsaturated carboxylic acid compound (i.e., a polymer of an $\alpha,\beta$-unsaturated carboxylic acid compound crosslinked by a crosslinking agent, which may sometimes be referred to as a "crosslinked $\alpha,\beta$-unsaturated carboxylic acid compound polymer") included in the present disclosure shows specific parameter R, within the range of 1.0 to 1.6, as measured using a BG dispersion of the crosslinked polymer and a neutralized aqueous solution of the crosslinked polymer. The crosslinked polymer may sometimes be referred to as "the crosslinked polymer of the present disclosure."

The crosslinked polymer of the present disclosure is a polymer formed from an $\alpha,\beta$-unsaturated carboxylic acid compound, as described above, and is crosslinked by a crosslinking agent. The crosslinked polymer is typically used as a hydrophilic thickener in the form of polymer particles. Adding these polymer particles to water or an aqueous solution containing water allows a particulate swollen gel to form, and neutralization causes the aqueous solution to thicken. A hydrophilic thickener containing the crosslinked polymer of the present disclosure may sometimes be referred to as "the thickener of the present disclosure."

Examples of $\alpha,\beta$-unsaturated carboxylic acid compounds include acrylic acid, methacrylic acid, crotonic acid, maleic acid, fumaric acid, itaconic acid, and salts thereof. Examples of the salts include sodium salts and potassium salts. Of these, (meth)acrylic acid (i.e., methacrylic acid and/or acrylic acid) and salts thereof are more preferred. These $\alpha,\beta$-unsaturated carboxylic acid compounds may be used alone or in a combination of two or more.

The crosslinking agent is preferably a compound having two or more ethylenically unsaturated groups. More specifically, examples of crosslinking agents include acrylic acid esters with two or more substitutions of a polyol, such as ethylene glycol, diethylene glycol, propylene glycol, polyoxyethylene glycol, polyoxypropylene glycol, glycerin, polyglycerin, trimethylolpropane, pentaerythritol, saccharose, and sorbitol; methacrylic acid esters with two or more substitutions of these polyols; allyl ethers with two or more substitutions of these polyols; diallyl phthalate, triallyl phosphate, allyl methacrylate, tetraallyloxyethane, triallyl cyanurate, divinyl adipate, vinyl crotonate, 1,5-hexadiene, divinylbenzene, etc. Of these, pentaerythritol triallyl ether, pentaerythritol tetraallyl ether, diethylene glycol diallyl ether, and polyarylsaccharose are more preferred. These crosslinking agents may be used alone or in a combination of two or more.

The crosslinked polymer of the present disclosure is preferably a polymer comprising about 0.15 to 2 parts by mass of the crosslinking agent per 100 parts by mass of the $\alpha,\beta$-unsaturated carboxylic acid compound. The upper or lower limit of the range (0.15 to 2 parts by mass) may be 0.2, 0.3, 0.4, 0.5, 0.6, 0.7, 0.8, 0.9, 1, 1.1, 1.2, 1.3, 1.4, 1.5, 1.6, 1.7, 1.8, or 1.9 parts by mass. For example, the range may be, for example, 0.4 to 1.5 parts by mass.

As described above, the specific parameter R of the crosslinked polymer of an $\alpha,\beta$-crosslinked unsaturated carboxylic acid compound is measured by using a BG dispersion of the crosslinked polymer and a neutralized aqueous solution of the crosslinked polymer. As described above, the crosslinked polymer of the present disclosure has a parameter R of 1.0 to 1.6. The upper or lower limit of the range may be, for example, 1.05, 1.1, 1.15, 1.2, 1.25, 1.3, 1.35, 1.4, 1.45, 1.5, or 1.55. The range may be, for example, 1.05 to 1.55.

Parameter R is the ratio of S1 to S2 (S1/S2) as calculated according to the following formula:

$$S1 = (k[\eta]_{aq}/k[\eta]_{BG})^{1/3}$$

$$S2 = (\text{particle size}_{aq}/\text{particle size}_{BG})$$

wherein

$k[\eta]_{BG}$ represents a product of interaction coefficient $k_{BG}$ and intrinsic viscosity $[\eta]_{BG}$, as determined by measuring a solution of the crosslinked polymer in 1,3-butylene glycol having an initial concentration of 0.3 mass% with an Ubbelohde dilution viscometer,

$k[\eta]_{aq}$ represents a product of interaction coefficient $k_{aq}$ and intrinsic viscosity $[\eta]_{aq}$, as determined by measuring a neutralized aqueous solution of the crosslinked polymer having an initial concentration of 0.015 mass% with an Ubbelohde dilution viscometer,

particle size$_{BG}$ represents a volume average particle size ($\mu$m) of the crosslinked polymer in a 0.3 mass% 1,3-butylene glycol solution, and

particle size$_{aq}$ represents a volume average particle size ($\mu$m) of the crosslinked polymer in a 0.1 mass% neutralized aqueous solution.

Intrinsic viscosity $[\eta]$ and interaction coefficient k of the crosslinked polymer solution are more specifically determined as follows. Specifically, flow time (t) of the solution and flow time ($t_0$) of the solvent alone are measured with an Ubbelohde dilution viscometer in accordance with JIS K-7367-1, and $\eta_{sp}$ is determined according to the following relational expression. $\eta_{sp}$ is a specific viscosity (indicating the rate of increase in viscosity caused by a solute in a solvent).

$$\eta_{sp} = (\eta - \eta_0)/\eta_0 = (\rho t - \rho_0 t_0)/\rho_0 t_0 \approx (t - t_0)/t_0 \ (\rho_0: \text{solvent viscosity}, t_0: \text{solvent flow time}, \rho: \text{solution viscosity}, t: \text{solution flow time})$$

While the concentration is changed by dilution, measurement is performed at four or more points (e.g., 4, 5, or 6 points), and the results are plotted with concentration (c) on the horizontal axis and ($\eta_{sp}/c$) on the vertical axis. Martin's equation $\eta_{sp}/c = [\eta]e^{k[\eta]c}$ with exponential approximation gives $[\eta]$ and k. (Plotting the measured values gives exponential approximation in the Martin's equation. The intercept of the extrapolation of zero concentration is intrinsic viscosity $[\eta]$.) Unless otherwise noted in the present specification, the unit for intrinsic viscosity $[\eta]$ is dl/g.

S1 satisfies the range of values for parameter R above and is preferably about 2.5 to 3.2. The upper or lower limit of the range may be, for example, 2.55, 2.6, 2.65, 2.7, 2.75, 2.8, 2.85, 2.9, 2.95, 3, 3.05, 3.1, or 3.15. The range may be, for example, 2.55 to 3.15.

$K[\eta]_{BG}$ satisfies the range of values for S1 above and is preferably about 1.5 to 2.3. The upper or lower limit of the range may be, for example, 1.55, 1.6, 1.65, 1.7, 1.75, 1.8, 1.85, 1.9, 1.95, 2, 2.05, 2.1, 2.15, 2.2, or 2.25. The range may be, for example, 1.55 to 2.25.

$K[\eta]_{aq}$ satisfies the range of values for S1 above and is preferably about 35 to 55. The upper or lower limit of the range may be, for example, 36, 37, 38, 39, 40, 41, 42, 43, 44, 45, 46, 47, 48, 49, 50, 51, 52, 53, or 54. The range may be, for example, 36 to 54.

The volume average particle size is determined according to laser diffractometry. For example, the volume average particle size can be determined with a laser diffraction particle size distribution analyzer (Shimadzu Corporation, model: SALD-2300).

S2 satisfies the range of values for parameter R and is preferably about 2 to 2.5. The upper or lower limit of the range may be, for example, 2.05, 2.1, 2.15, 2.2, 2.25, 2.3, 2.35, 2.4, or 2.45. The range may be, for example, 2.05 to 2.45.

A 0.5 mass% neutralized aqueous solution of the crosslinked polymer of the present disclosure at 25°C preferably has a yield stress of 20 Pa or lower, and preferably 1 to 20 Pa. The upper or lower limit of the range may be, for example, 2, 3, 4, 5, 6, 7, 8, 9, 10, 11, 12, 13, 14, 15, 16, 17, 18, or 19 Pa. For example, the range may be 5 to 19 Pa.

The yield stress is measured with a rheometer. For example, the yield stress is measured by using a dynamic viscoelasticity analyzer (AR-2000ex from TA Instruments Japan) with a 60-mm (diameter), 4-degree cone. More specifically, the steady-state flow viscosity at a shear stress of $1.0 \times 10^{-5}$ to $1.0 \times 10^3$ (Pa) is measured under steady-state conditions that satisfy the following three items: (1) percentage tolerance 1.0, (2) consecutive within tolerance 3, and (3) maximum point time 10 min. The point at which the strain changes abruptly with respect to shear stress is determined to be the yield value (yield stress, Pa).

The neutralized aqueous solution of the crosslinked polymer used for the measurement is a 0.5 mass% neutralized aqueous solution of the polymer, as described above. For example, the neutralized aqueous solution of the crosslinked polymer is prepared as follows. 288.8 g of ion-exchanged water is placed in a 500-mL beaker, and 1.5 g of a crosslinked polymer of an $\alpha,\beta$-unsaturated carboxylic acid compound is gradually added with stirring at 1500 rpm with a dispersion mixer. After subsequent stirring for 30 minutes, the dispersion mixer is turned off, and 9.7 g of a 6 mass% aqueous sodium hydroxide solution is added and stirred with a handheld mixer for 30 seconds to obtain a 0.5 mass% neutralized aqueous solution of the crosslinked polymer.

An aqueous solution with a pH of 7 (measured with a pH meter at 25°C) is designated as a neutralized aqueous solution. A pH adjuster can be used for neutralization; for example, sodium hydroxide is suitable as described above. The same applies below.

The crosslinked polymer of the present disclosure can be obtained by reacting an $\alpha,\beta$-unsaturated carboxylic acid compound with a crosslinking agent in an inert solvent at a specific temperature in the presence of a radical polymerization catalyst.

The amount of the $\alpha,\beta$-unsaturated carboxylic acid compound is, for example, preferably 6 to 25 parts by volume, more preferably 8 to 22 parts by volume, and still more preferably 13 to 20 parts by volume, per 100 parts by volume of the inert solvent.

Examples of radical polymerization catalysts include, but are not particularly limited to, azobisisobutyronitrile, 2,2'-azobis-2,4-dimethylvaleronitrile, dimethyl-2,2'-azobisisobutyrate, benzoyl peroxide, lauroyl peroxide, cumene hydroperoxide, and tert-butyl hydroperoxide. These radical polymerization catalysts may be used alone or in a combination of two or more. In particular, 2,2'-azobis-2,4-dimethylvaleronitrile is preferably used from the viewpoint of its suitably exhibited catalytic activity at a reaction temperature. The amount of the radical polymerization catalyst is, for example, preferably about 0.01 to 0.45 parts by mass, and more preferably about 0.01 to 0.35 parts by mass, per 100 parts by mass of the $\alpha,\beta$-unsaturated carboxylic acid compound.

The inert solvent is a solvent that dissolves an $\alpha,\beta$-unsaturated carboxylic acid compound and a crosslinking agent, but does not dissolve the resulting crosslinked polymer of the $\alpha,\beta$-unsaturated carboxylic acid compound. More specifically, examples of such solvents include n-pentane, n-hexane, isohexane, n-heptane, n-octane, isooctane, cyclopentane, methylcyclopentane, cyclohexane, methylcyclohexane, benzene, toluene, xylene, chlorobenzene, ethylene dichloride, ethyl acetate, isopropyl acetate, methyl ethyl ketone, and methyl isobutyl ketone. These may be used alone or in a combination of two or more. In particular, n-hexane is preferred from the viewpoint of its suitably exhibited catalytic activity at a reaction temperature.

The atmosphere for the reaction is, for example, preferably an inert gas atmosphere such as nitrogen gas or an argon gas.

The reaction is performed at relatively lower temperatures than in conventional methods. The reaction performed at relatively lower temperatures than in conventional methods preferably gives the crosslinked polymer of the present disclosure with parameter R within the range of 1.0 to 1.6. For example, the reaction is preferably performed at about 40 to 48°C. The upper or lower limit of the temperature range may be, for example, 41, 42, 43, 44, 45, 46, or 47°C. For example, the temperature range may be 43 to 47°C. The reaction time can be set as appropriate, preferably to about 2 to 10 hours, and more preferably about 3 to 7 hours. Polymerization can preferably be performed, for example, by precipitation polymerization.

After the reaction is complete, the reaction solution can be heated to, for example, about 80 to 130°C to volatilize and remove the inert solvent. This gives a white fine powder of a crosslinked carboxyl group-containing polymer.

As described above, the crosslinked polymer of the present disclosure can preferably be used as a thickener (in particular, a hydrophilic thickener). The present disclosure also preferably encompasses thickeners containing the crosslinked polymer of the present disclosure. In particular, such thickeners can preferably be used for preparing an external composition. Preferred examples of external compositions include pharmaceutical compositions and cosmetic compositions. The present disclosure also preferably encompasses external compositions containing the crosslinked polymer of the present disclosure. Such external compositions may sometimes be referred to as "the external composition of the present disclosure."

The content of the crosslinked polymer of the present disclosure in the external composition of the present disclosure is preferably 1.0 mass% or lower, and more preferably 0.9, 0.8, 0.7, 0.6, or 0.5 mass% or lower. The lower limit of the content is, for example, but is not particularly limited to, 0.01 mass% or 0.05 mass%.

In the present specification, the term "comprising" also includes the concepts of "consisting essentially of" and "consisting of." The present disclosure also encompasses every combination of the technical elements described in the specification.

The various characteristics (properties, structures, functions, etc.) explained for each embodiment of the present disclosure described above may be combined in any way in identifying the subject matter encompassed by the present disclosure. In other words, the present disclosure encompasses all subject matter of every possible combination of the characteristics described in the present specification.

Examples

The following describes in more detail embodiments of the present disclosure with reference to the following Examples. However, the embodiments of the present disclosure are not limited to these Examples.

Production of Crosslinked Polymer Particles of $\alpha,\beta$-unsaturated Carboxylic Acid Compound

Example 1

Production Method

A 500-mL round separable flask was prepared as a reaction vessel, and 35 g (33 mL) of acrylic acid and 0.35 g of 2,2'-azobis(2,4-dimethylvaleronitrile) were placed in the flask. Further, a solution of 0.24 g of pentaerythritol tetraallyl ether in 248 g (366 mL) of n-hexane was added gently. The separable flask containing the solution was fixed to a four-necked separable cover equipped with a stirrer, a thermometer, a glass tube for nitrogen feeding, and a cooling tube. An agitator was equipped with four inclined paddle blades and an anchor-shaped agitator blade, and the inclined paddle blades were a combination of blades with different lengths.

While the solution prepared in the reaction vessel was stirred and mixed uniformly, nitrogen gas was blown into the solution at room temperature to deoxidize the upper space of the reaction vessel and the solution. After sufficient deoxidization, while the internal temperature of the solution was kept at 44 to 46°C, the slurry generated in the reaction was allowed to react in a nitrogen atmosphere for 4 hours with stirring at a peripheral speed of 0.075 to 0.175 m/min. After the reaction was complete, the generated white slurry was heated to 95 to 105°C to remove n-hexane, and then dried under reduced pressure at 120°C at 4 to 5 mmHg for 8 hours, thereby obtaining 33 g of a white fine powder of a crosslinked polymer of an $\alpha,\beta$-unsaturated carboxylic acid compound.

Example 2

Production Method

The operation of the production method of Example 1 was performed in the same manner, except that 45 g (43 mL) of acrylic acid, 0.45 g of 2,2'azobis(2,4-dimethylvaleronitrile), and 0.25 g of pentaerythritol tetraallyl ether were used, thereby obtaining 43 g of a white fine powder of a crosslinked polymer of an $\alpha,\beta$-unsaturated carboxylic acid compound.

Example 3

Production Method

The operation of the production method of Example 1 was performed in the same manner, except that 57 g (55 mL) of acrylic acid, 0.46 g of 2,2'-azobis(2,4-dimethylvaleronitrile), and 0.48 g of pentaerythritol tetraallyl ether were used, thereby obtaining 50 g of a white fine powder of a crosslinked polymer of an $\alpha,\beta$-unsaturated carboxylic acid compound.

Comparative Example 1

Production Method

63 g (60 mL) of acrylic acid, 0.44 g of pentaerythritol tetraallyl ether, 0.016 g of azobisisobutyronitrile, and 375 g (300 mL) of ethylene dichloride were placed in a 500-mL four-necked flask equipped with a stirrer, a thermometer, a nitrogen-feeding tube, and a cooling tube. Subsequently, the mixture was uniformly stirred and mixed, and then nitrogen gas was blown into the solution to remove oxygen present in the upper space of the reaction vessel, in the starting materials, and in the solvent. The mixture was then allowed to react for 3 hours while being kept at 70 to 75°C in a nitrogen atmosphere. After the reaction was complete, the generated slurry was heated to 110°C to distill ethylene dichloride, thereby obtaining 63 g of a white fine powder of a crosslinked polymer of an $\alpha,\beta$-unsaturated carboxylic acid compound.

Comparative Example 2

Production Method

The operation of the production method of Comparative Example 1 was performed in the same manner, except that 44 g (42 mL) of acrylic acid, 0.22 g of lauroyl peroxide, and 0.31 g of pentaerythritol triallyl ether were used, and the reaction time was 6 hours, thereby obtaining 40 g of a white fine powder of a crosslinked polymer of an $\alpha,\beta$-unsaturated carboxylic acid compound.

Comparative Example 3

Production method

The operation of the production method of Comparative Example 1 was performed in the same manner, except that 44 g (42 mL) of acrylic acid, 0.18 g of lauroyl peroxide, and 0.22 g of pentaerythritol triallyl ether were used, and the reaction time was 6 hours, thereby obtaining 41 g of a white fine powder of a crosslinked polymer of an $\alpha,\beta$-unsaturated carboxylic acid compound.

Evaluation of Physical Properties of Crosslinked Polymer Particles of $\alpha,\beta$-Unsaturated Carboxylic Acid Compound Measurement with Ubbelohde Dilution Viscometer

A 0.3 mass% solution of the crosslinked polymer particles of an $\alpha,\beta$-unsaturated carboxylic acid compound obtained in the individual Examples in 1,3-butylene glycol (1 mM lithium chloride) was prepared. More specifically, 297 g of 1,3-butylene glycol was placed in a 500-mL beaker. With stirring at 1500 rpm with a dispersion mixer, 3.0 g of crosslinked polymer particles of an $\alpha,\beta$-unsaturated carboxylic acid compound were gradually added, and the mixture was further stirred for 30 minutes, followed by stirring the mixture in a water bath at 60 to 80°C for 24 hours. After stirring, suction filtration was performed by using a 25-pm filter cloth, thereby obtaining a 2.0 mass% solution of the polymer in 1,3-butylene glycol. This polymer solution was then diluted with BG so that the concentration of the polymer was 0.3 mass%, and lithium chloride was also added to give a concentration of 1 mM.

The obtained 0.3 mass% BG solution of the polymer was measured for flow time at 30.0 ± 0.01°C with an Ubbelohde dilution viscometer. The Ubbelohde dilution viscometer for use was one with a flow time of 100 to 200 seconds during solvent measurement in accordance with JIS K-7367-1 (Asahi Glassplant Inc. 2B (Cat. No. 4804-09)). The measurement method was performed in accordance with JIS K-7367-1. More specifically, the flow time of the 0.3 mass% solution was first measured, and then a predetermined amount of a solvent was further added to dilute the 0.3 mass% solution, followed by measuring the flow time. This operation was performed several times (about 4 to 5 times) to measure the flow time at each concentration. With flow time (t) at each concentration and solvent-only flow time ($t_0$), $\eta_{sp}$ can be determined according to the following relational expression. $\eta_{sp}$ is a specific viscosity (indicating the rate of increase in viscosity caused by a solute in a solvent).

$$\eta_{sp} = (\eta - \eta_0)/\eta_0 = (\rho t - \rho_0 t_0)/\rho_0 t_0 \approx (t - t_0)/t_0 \ (\rho_0: \text{solvent viscosity}, t_0: \text{solvent flow time}, \rho: \text{solution viscosity}, t: \text{solution flow time})$$

Then, the results are plotted with concentration (c) on the horizontal axis and ($\eta_{sp}/c$) on the vertical axis. Martin's equation $\eta_{sp}/c = [\eta]e^{k[\eta]c}$ with exponential approximation gives $[\eta]$ and k. (Plotting the measured values gives exponential approximation in the Martin's equation. The intercept of the extrapolation of zero concentration is intrinsic viscosity $[\eta]$.) $[\eta]$ denotes an intrinsic viscosity, k denotes an interaction coefficient, and $k[\eta]$ denotes the product of these. The unit for $[\eta]$ is dl/g.

The intrinsic viscosity of the BG solution of the polymer may be denoted as $[\eta]_{BG}$, the interaction coefficient may be denoted as $k_{BG}$, and the product of these may be denoted as $k[\eta]_{BG}$. The intrinsic viscosity of the neutralized aqueous solution of the polymer may be denoted as $[\eta]_{aq}$, the interaction coefficient may be denoted as $k_{aq}$, and the product of these may be denoted as $k[\eta]_{aq}$.

A 0.015 mass% neutralized aqueous solution of crosslinked polymer particles of an $\alpha,\beta$-unsaturated carboxylic acid compound was also prepared. More specifically, 288.8 g of ion-exchanged water was placed in a 500-mL beaker, and 1.5 g of crosslinked carboxyl group-containing polymer particles were gradually added with stirring at 1500 rpm with a dispersion mixer. After subsequent stirring for 30 minutes, the dispersion mixer was turned off, and 9.7 g of a 6 mass% aqueous sodium hydroxide solution was added, followed by stirring the mixture for 30 seconds with a handheld mixer, thereby obtaining a 0.5 mass% neutralized aqueous solution of crosslinked carboxyl group-containing polymer particles. The neutralized aqueous solution was then further diluted with ion-exchanged water to 0.015 mass%. The 0.015 mass% neutralized aqueous solution was measured with an Ubbelohde dilution viscometer at 25.0 ± 0.01°C. The solution was successively diluted in the same manner as above and measured in the same manner. The results of measurement with the Ubbelohde dilution viscometer were plotted to determine the value of $k[\eta]_{aq}$ according to the Martin's equation.

Then, the following definition was made: $S1 = (k[\eta]_{aq}/k[\eta]_{BG})^{1/3}$.

$K[\eta]$ is considered to be an index reflecting the spread of crosslinked carboxyl group-containing polymer particles in each solution. If $k[\eta]$ is small, the spread in a solution is small, whereas if $k[\eta]$ is large, the spread in a solution is large. Thus, S1, which is the ratio of the spread in the BG solution to the spread in the neutralized aqueous system, can be considered to be the index of spread of the polymer particles in a solution.

Particle Size Measurement

Crosslinked polymer particles of an $\alpha,\beta$-unsaturated carboxylic acid compound were prepared into a 0.1 mass% neutralized aqueous solution and a 0.3 mass% 1,3-butylene glycol solution. The particle size distribution and volume average particle size (median diameter) of the polymer particles in each of these solutions were measured with a laser diffraction particle size distribution analyzer (Shimadzu Corporation, model: SALD-2300).

The ratio of the volume average particle size in the neutralized aqueous solution to the volume average particle size in the 1,3-butylene glycol solution (particle size_aq/particle size_BG) was determined to be S2.

Further, the following definition was made: R = (S1/S2).

Yield Value Measurement

The viscosity of a 0.5 mass% neutralized aqueous solution of crosslinked polymer particles of an $\alpha,\beta$-unsaturated carboxylic acid compound was measured with a dynamic viscoelasticity analyzer (rheometer: AR-2000ex from TA Instruments). The steady-state flow viscosity at a shear stress of $1.0 \times 10^{-5}$ to $1.0 \times 10^{3}$ (Pa) was measured by using a 60-mm (diameter), 4-degree cone with the temperature set to 25°C under steady-state conditions that satisfied the following three items: (1) percentage tolerance 1.0, (2) consecutive within tolerance 3, and (3) maximum point time 10 min. The point at which the strain changed abruptly with respect to shear stress was determined to be the yield value (yield stress, Pa).

Table 1 summarizes the results.

Texture Evaluation

288.8 g of ion-exchanged water was placed in a 500-mL beaker, and 1.5 g of crosslinked carboxyl group-containing polymer particles were gradually added with stirring at 1500 rpm with a dispersion mixer. After subsequent stirring for 30 minutes, the dispersion mixer was turned off, and 9.7 g of a 6 mass% aqueous sodium hydroxide solution was added and stirred with a handheld mixer for 30 seconds to obtain a 0.5 mass% neutralized aqueous solution of crosslinked carboxyl group-containing polymer particles.

The resulting neutralized aqueous solutions were evaluated for texture. Ten panelists, including men and women, were selected as evaluators. The panelists applied and spread each neutralized aqueous solution on the back of their hands. The scores of the panelists who felt that the texture of the spread aqueous solution was good (refreshing sensation, non-stickiness, lightness, moist texture, and ease of spreading) were totaled (five-level evaluation indicated below). The average value was determined to be the score for each evaluation item. In general, an average value of 4 or higher can be considered excellent.

Evaluation Criteria

> 5: Very good
> 4: Good
> 3: Average
> 2: Fair
> 1: Poor

Table 2 shows the results of texture evaluation.

Table 1

| | 1,3-Butylene Glycol Solution | | | Neutralized Aqueous Solution | | | | | | |
| | Ubbelohde Dilution Viscometer | | SALD-2300 | Rheometer | Ubbelohde Dilution Viscometer | | SALD-2300 | | | |
| | $[\eta]_{BG}$ | $k[\eta]_{BG}$ | Average Particle Size (μm) | Yield Value (Pa) | $[\eta]_{aq}$ | $k[\eta]_{aq}$ | Average Particle Size (μm) | S1 | S2 | R |
|---|---|---|---|---|---|---|---|---|---|---|
| Example 1 | 1.5 | 2.1 | 8.3 | 13 | 30.7 | 37.6 | 19.6 | 2.60 | 2.36 | 1.10 |
| Example 2 | 1.5 | 1.9 | 9.5 | 12 | 25.5 | 40.9 | 22.0 | 2.78 | 2.32 | 1.20 |
| Example 3 | 1.5 | 1.7 | 19.0 | 17 | 25.7 | 53.4 | 40.7 | 3.16 | 2.14 | 1.47 |
| Comparative Example 1 | 1.5 | 1.9 | 7.2 | 14 | 23.3 | 39.8 | 11.0 | 2.77 | 1.53 | 1.81 |
| Comparative Example 2 | 1.5 | 1.9 | 6.2 | 20 | 26.8 | 45.5 | 8.8 | 2.86 | 1.42 | 2.02 |
| Comparative Example 3 | 1.5 | 1.5 | 7.7 | 15 | 30.4 | 48.1 | 10.6 | 3.16 | 1.37 | 2.30 |

Table 2

| | Texture Evaluation | | | | |
|---|---|---|---|---|---|
| | Refreshing Sensation | No Stickiness | Lightness | Moist Texture | Ease of Spreading |
| Example 1 | 5 | 4 | 4.5 | 4 | 4 |
| Example 2 | 5 | 3 | 3 | 5 | 4 |
| Example 3 | 4.5 | 4 | 4 | 4.5 | 4 |
| Comparative Example 1 | 4 | 3 | 2.5 | 4 | 4 |
| Comparative Example 2 | 3 | 3 | 2 | 3.5 | 3 |
| Comparative Example 3 | 2.5 | 3 | 3 | 4 | 3 |

The results indicate that the neutralized aqueous solutions of crosslinked carboxyl group-containing polymer particles obtained in Examples 1 to 3 had an excellent texture (especially in refreshing sensation), and that the polymer is useful, for example, as a thickener for cosmetics. The results also indicate that a relatively low R results in relatively high texture evaluation values. Fig. 1 is a graph showing the relation between value R and refreshing sensation. In Fig. 1, "•" indicates the results of the Examples, whereas "▲" indicates the results of the Comparative Examples.

## Claims

1. A crosslinked polymer of an $\alpha,\beta$-unsaturated carboxylic acid compound, the polymer being crosslinked by a crosslinking agent,
wherein

   the crosslinked polymer has a ratio of S1 to S2 (S1/S2) within the range of 1.0 to 1.6 as calculated according to the following formulas:

   $$S1 = (k[\eta]_{aq}/k[\eta]_{BG})^{1/3}$$

   $$S2 = (particle\ size_{aq}/particle\ size_{BG})$$

   wherein

   $k[\eta]_{BG}$ represents a product of interaction coefficient $k_{BG}$ and intrinsic viscosity $[\eta]_{BG}$, as determined by measuring a solution of the crosslinked polymer in 1,3-butylene glycol having an initial concentration of 0.3 mass% with an Ubbelohde dilution viscometer,
   $k[\eta]_{aq}$ represents a product of interaction coefficient $k_{aq}$ and intrinsic viscosity $[\eta]_{aq}$, as determined by measuring a neutralized aqueous solution of the crosslinked polymer having an initial concentration of 0.015 mass% with an Ubbelohde dilution viscometer,
   particle $size_{BG}$ represents a volume average particle size ($\mu$m) of the crosslinked polymer in a 0.3 mass% 1,3-butylene glycol solution, and
   particle $size_{aq}$ represents a volume average particle size ($\mu$m) of the crosslinked polymer in a 0.1 mass% neutralized aqueous solution.

2. The crosslinked polymer according to claim 1, wherein a 0.5 mass% neutralized aqueous solution of the crosslinked polymer at 25°C has a yield stress of 20 Pa or lower.

3. The crosslinked polymer according to claim 1, wherein the $\alpha,\beta$-unsaturated carboxylic acid compound is at least one member selected from the group consisting of acrylic acid, methacrylic acid, crotonic acid, maleic acid, fumaric acid, itaconic acid, and salts thereof.

4. The crosslinked polymer according to claim 1, wherein the crosslinking agent is a compound having two or more ethylenically unsaturated groups.

5. The crosslinked polymer according to claim 1, which is a polymer comprising 0.15 to 2 parts by mass of the crosslinking agent per 100 parts by mass of the $\alpha,\beta$-unsaturated carboxylic acid compound.

6. The crosslinked polymer according to claim 1, wherein S2 is 2 to 2.5.

7. A thickener for preparing an external composition, the thickener comprising the crosslinked polymer of any one of claims 1 to 6.

8. An external composition comprising the crosslinked polymer of any one of claims 1 to 6.

9. A method for producing a polymer of an $\alpha,\beta$-unsaturated carboxylic acid compound crosslinked by a crosslinking agent, comprising reacting an $\alpha,\beta$-unsaturated carboxylic acid compound and a compound having two or more ethylenically unsaturated groups in an inert solvent at 40 to 48°C in the presence of a radical polymerization catalyst.

Fig. 1

## INTERNATIONAL SEARCH REPORT

| International application No. |
| --- |
| **PCT/JP2022/039822** |

### A. CLASSIFICATION OF SUBJECT MATTER

*C08F 222/02*(2006.01)i
FI: C08F222/02

According to International Patent Classification (IPC) or to both national classification and IPC

### B. FIELDS SEARCHED

Minimum documentation searched (classification system followed by classification symbols)

C08F222/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2-22312 A (SEITETSU KAGAKU CO., LTD.) 25 January 1990 (1990-01-25) <br> claims, p. 2, upper left column, Industrial application field, p. 2, lower right column, lines 6-14, examples | 1-8 |
| X | WO 2019/244952 A1 (SUMITOMO SEIKA CHEMICALS CO., LTD.) 26 December 2019 (2019-12-26) <br> paragraph [0085] | 1-6 |
| A | JP 2005-120131 A (SUMITOMO SEIKA CHEMICALS CO., LTD.) 12 May 2005 (2005-05-12) <br> entire text | 1-8 |
| A | WO 2020/179602 A1 (SUMITOMO SEIKA CHEMICALS CO., LTD.) 10 September 2020 (2020-09-10) <br> entire text | 1-8 |

☐ Further documents are listed in the continuation of Box C.   ☑ See patent family annex.

| | |
| --- | --- |
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **27 December 2022** | **10 January 2023** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT

| International application No. |
| --- |
| **PCT/JP2022/039822** |

**Box No. III    Observations where unity of invention is lacking (Continuation of item 3 of first sheet)**

This International Searching Authority found multiple inventions in this international application, as follows:

Claims are classified into the two inventions below.
(Invention 1) Claims 1-8
    Claims 1-8 have the special technical feature of a "cross-linked polymer of an α,β-unsaturated carboxylic acid compound crosslinked by a crosslinking agent, wherein a specific parameter S1/S2 is 1.0-1.6," and are thus classified as invention 1.

(Invention 2) Claim 9
    Claim 9 shares, with claim 1 classified as invention 1, the common technical feature of a "polymer of an α,β-unsaturated carboxylic acid compound crosslinked by a crosslinking agent." However, said technical feature is well-known that it is not necessary to cite documents, and thus cannot be considered a special technical feature. Also, there are no other identical or corresponding special technical features between claim 9 and claim 1.
    In addition, claim 9 is not dependent on claim 1. Furthermore, claim 9 is not substantially identical to or similarly closely related to any of the claims classified as invention 1.
    Accordingly, claim 9 cannot be classified as invention 1.
    In addition, claim 9 has the special technical feature of a "method for producing a polymer of an α,β-unsaturated carboxylic acid compound crosslinked by a crosslinking agent, the method comprising reacting an α,β-unsaturated carboxylic acid compound with a compound having two or more ethylenically unsaturated groups in an inert solvent at 40-48 °C in the presence of a radical polymerization catalyst," and is thus classified as invention 2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-8**

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
Information on patent family members

International application No.

**PCT/JP2022/039822**

| Patent document cited in search report | | | Publication date (day/month/year) | Patent family member(s) | | | Publication date (day/month/year) |
|---|---|---|---|---|---|---|---|
| JP | 2-22312 | A | 25 January 1990 | EP 343490 A2 claims, p. 2, lines 4-17, p. 2, line 52 to p. 3, line 1, examples | | | |
| WO | 2019/244952 | A1 | 26 December 2019 | US 2021/0130742 A1 paragraph [0123] EP 3812423 A1 CN 112041390 A KR 10-2021-0022531 A | | | |
| JP | 2005-120131 | A | 12 May 2005 | (Family: none) | | | |
| WO | 2020/179602 | A1 | 10 September 2020 | US 2022/0054391 A1 entire text EP 3932962 A1 CN 113508145 A KR 10-2021-0136015 A | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 4883844 A
- WO 5943798 A
- WO 6182975 A
- WO 4630101 A